# EUROPÄISCHE PATENTANMELDUNG

(11) **EP 1 514 865 A1**
(43) Veröffentlichungstag der Anmeldung: **16.03.2005**
(21) Anmeldenummer: 04020500.7
(22) Anmeldetag: 28.08.2004
(51) Int. Cl.: C07C 215/76, C07C 217/82, C07C 245/08

(54) **Verfahren zur Herstellung von Halogenhaltigen 4-Aminophenolen**

(30) Priorität: 09.09.2003 DE 10341533
(71) Anmelder: Bayer Chemicals AG, 51368 Leverkusen (DE)
(72) Erfinder: Peilstöcker, Karen, Dr., 51061 Köln (DE); Marhold, Albrecht, Dr., 51373 Leverkusen (DE); Joschek, Jens-Peter, Dr., 51061 Köln (DE)

(57) **Zusammenfassung**

Die vorliegende Erfindung betrifft halogenierte 4-Aminophenole sowie halogenierte 4-(Phenyldiazenyl)phenole, ein Verfahren zu deren Herstellung sowie die Anwendung der halogenierten 4-Hydroxyphenole zur Herstellung von Wirkstoffen insbesondere in Arzneimitteln und Agrochemikalien.

## Beschreibung

Die vorliegende Erfindung betrifft halogenierte 4-Aminophenole sowie halogenierte 4-(Phenyldiazenyl)phenole, ein Verfahren zu deren Herstellung sowie die Anwendung der halogenierten 4-Hydroxyphenole zur Herstellung von Wirkstoffen insbesondere in Arzneimitteln und Agrochemikalien.

Halogenierte Phenole, wie insbesondere fluorhaltige Phenole sind wertvolle Ausgangsprodukte für die Herstellung von Wirkstoffen in Arzneimitteln und Agrochemikalien, da die Fluorsubstituenten die Lipophilie und damit die Membrangängigkeit des gesamten Wirkstoffmoleküls erhöhen. So eignen sich beispielsweise fluorhaltige 4-Hydroxyaniline besonders als Ausgangsprodukte zur Herstellung von Arzneimitteln, die zur Behandlung von chronischer Bronchitis eingesetzt werden (siehe auch WO 03/08413 und PCT/03/02154).

Die Herstellung beispielsweise von 2,5-Difluor-4-hydroxyanilin erfolgt üblicherweise durch Nitrierung von 2,5-Difluorphenol und anschließende Reduktion (siehe JP-A 63 310850).

Nachteilig an dieser Syntheseroute ist, dass die Nitrierungsreaktion nicht selektiv verläuft und das als Intermediat gewünschte 4-Nitrophenol nur in schlechten Ausbeuten erhältlich ist.

Es bestand daher das Bedürfnis, ein Verfahren bereitzustellen, das die Herstellung von halogenierten 4-Aminophenolen in guten Ausbeuten und in einfacher Weise ermöglicht.

Es wurde nun ein Verfahren zur Herstellung von Verbindungen der Formel (I) gefunden, 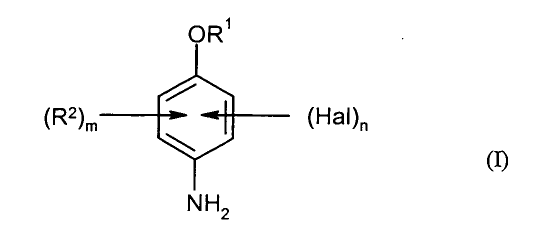 in der
- m: für 0, 1, 2 oder 3 steht und
- n: für 1, 2, 3 oder 4 steht,
wobei die Summe aus
- m + n: maximal 4 ist und
- R¹: für Wasserstoff, C₁-C₁₂-Alkyl oder C₅-C₁₅-Arylalkyl und
- R²: jeweils unabhängig für C₁-C₁₂-Fluoralkyl, C₁-C₁₂-Fluoralkylthio oder C₁-C₁₂-Fluoralkoxy und
- Hal: jeweils unabhängig für Brom, Chlor oder Fluor steht
das dadurch gekennzeichnet ist, dass
- in einem Schritt A1)
   Verbindungen der Formel (II) 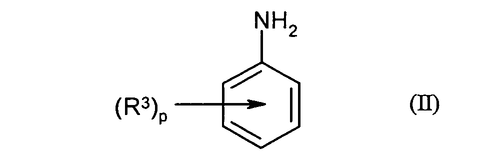 in der
   - p: für 0, 1, 2 oder 3 steht und
   - R³: jeweils unabhängig voneinander für Fluor, Chlor, Brom, Iod, Cyano, Thiocyanato, Hydroxysulfonyl oder Alkalimetallsalze davon, Nitro, C₁-C₁₂-Alkyl, C₁-C₁₂-Fluoralkyl, C₁-C₁₂-Fluoralkyoxy, C₁-C₁₂-Fluoralkylthio, C₁-C₁₂-Alkoxy, C₁-C₁₂-Alkoxycarbonyl, Di(C₁-C₁₂-alkyl)amino, C₄-C₁₄-Aryl oder C₅-C₁₅-Arylalkyl steht,
   in Diazoniumsalze der Formel (III) überführt werden 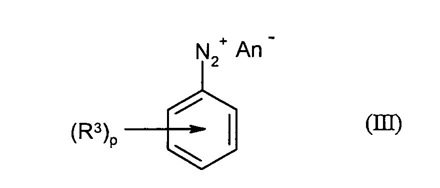 in der An⁻ für das Anion einer Säure steht
   und
- in einem Schritt A2)
   die Diazoniumsalze der Formel (III) mit Verbindungen der Formel (IV) 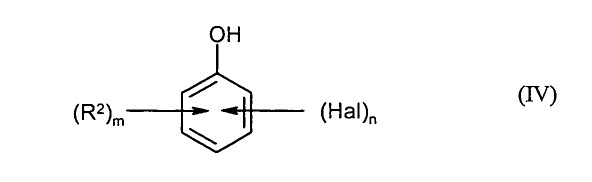 in der
   m, n, Hal und R² die unter der Formel (I) angegebene Bedeutung besitzen
   in Gegenwart von Base zu Verbindungen der Formel (V) umgesetzt werden 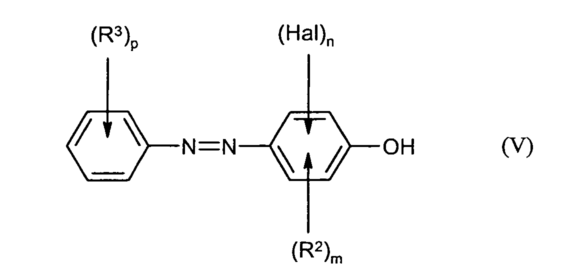 und
- in einem Schritt B) die Verbindungen der Formel (V) mit einem Reduktionsmittel in die Verbindungen der Formel (I) überführt werden, in der R¹ für Wasserstoff steht und
- gegebenenfalls in einem Schritt C) diese Verbindungen durch O-Alkylierung in Verbindungen der Formel (I) überführt werden, in der R¹ für C₁-C₁₂-Alkyl steht.

Ausgenommen ist dabei die Herstellung von 4-Amino-3,5-difluorphenol und 4-Amino-3-fluorphenol.

Gegebenenfalls können in einem Schritt D)
- die Verbindungen der Formel (I) durch Umsetzung mit Verbindungen der Formeln (VIIa) oder (VIIb) 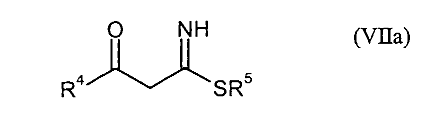 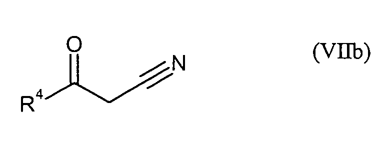 in denen
   - R⁴: für 2,4-Difluorphenyl, 4-Fluorphenyl, 2,3-Difluorphenyl oder 4-Fluor-3-chlorphenyl und
   - R⁵: für gegebenenfalls einfach oder mehrfach durch Chlor oder Fluor substituiertes Phenyl steht,
   in Verbindungen der Formel (VIII) überführt werden, 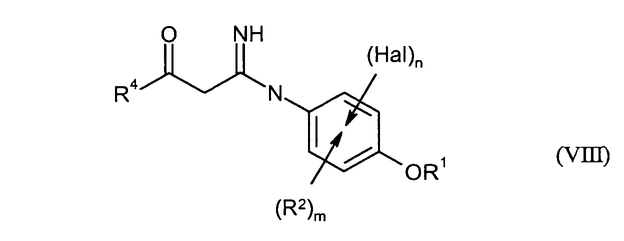 in der
   - R¹, R², R⁴, Hal, n und m: die obenstehend genannte Bedeutung besitzen.

Schritt D) kann dabei in an sich bekannter Weise z.B. analog wie in US 4,851,535 beschrieben durchgeführt werden.

Gegebenenfalls können in einem Schritt E)
- die Verbindungen der Formel (VIII) durch Umsetzung mit Verbindungen der Formel (IX) 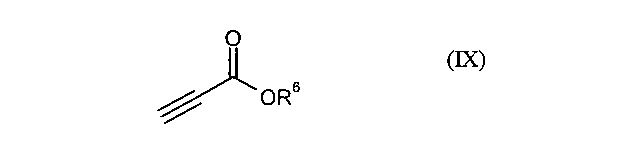 in der
   - R⁶: für C₁-C₁₂-Alkyl, C₅-C₁₅-Arylalkyl oder C₄-C₁₄-Aryl steht
   in Verbindungen der Formel (X) überführt werden, 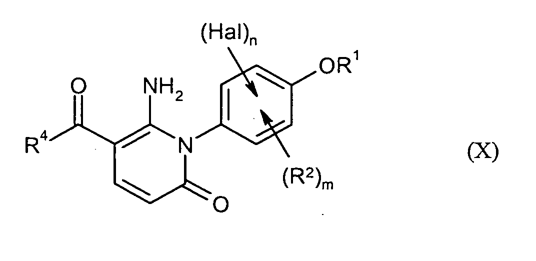 in der
   R¹, R², R⁴, R⁶, Hal, n und m die obenstehend genannte Bedeutung besitzen.

Die Umsetzung der Verbindungen der Formel (VIII) mit Verbindungen der Formel (IX) zu Verbindungen der Formel (X) kann beispielsweise durch erhitzen der beiden Reaktanden in einem organischen Lösungsmittel wie vorzugsweise einem aliphatischen Alkohol wie beispielsweise Methanol erfolgen.

Die für die Schritte A) bis C) als Ausnahmen genannten Verbindungen sind für die Schritte D) und E) in gleicher Weise ausgenommen.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**Alkyl** beziehungsweise Alkoxy steht jeweils unabhängig für einen geradkettigen, cyclischen, verzweigten oder unverzweigten Alkyl- beziehungsweise Alkoxy-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₁₂-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Nonyl, n-Decyl und n-Dodecyl.

**Fluoralkyl** beziehungsweise **Fluoralkoxy** beziehungsweise **Fluoralkylthio** bedeutet jeweils unabhängig einen geradkettigen, cyclischen, verzweigten oder unverzweigten Alkyl- beziehungsweise Alkoxy- beziehungsweise Alkylthio-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

Beispielsweise steht C₁-C₁₂-Fluoralkoxy für Trifluormethoxy, 2,2,2-Trifluorethoxy, Pentafluorethoxy, Nonafluorbutoxy, Heptafluorisopropoxy, Perfluoroctoxy und Perfluordodecoxy.

Beispielsweise steht C₁-C₁₂-Fluoralkylthio für Trifluormethylthio, 2,2,2-Trifluorethylthio, Pentafluorethylthio, Nonaflubrbutylthio, Heptafluorisopropylthio, Perfluoroctylthio und Perfluordodecylthio.

Aryl steht jeweils unabhängig für einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, oder vorzugsweise für einen carbocyclischen aromatischen Rest mit 6 bis 14 Gerüstkohlenstoffatomen.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Fluor, Cyano, Nitro, C₁-C₁₂-Alkyl, C₁-C₁₂-Fluoralkyl, C₁-C₁₂-Fluoralkoxy, C₁-C₁₂-Fluoralkylthio, C₁-C₁₂-Alkoxy, Di(C₁-C₁₂-alkyl)amino.

**Arylalkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Defmition substituiert sein kann.

Im Folgenden werden die bevorzugten Substitutionsmuster definiert:
- n: steht bevorzugt für 1 oder 2,
- m: steht bevorzugt für 0 oder 1,
- R¹: steht bevorzugt für Wasserstoff oder Methyl, besonders bevorzugt für Wasserstoff,
- R²: steht bevorzugt für Trifluormethyl, Trifluormethoxy und Trifluormethylthio,
- Hal: steht bevorzugt für Brom, Chlor oder Fluor, wobei weiter bevorzugt mindestens ein Rest Hal Fluor ist.

In Verbindungen der Formel (II) steht p vorzugsweise für 0.

Das erfindungsgemäße Verfahren eignet sich insbesondere zur Herstellung von 4-Amino-2,3-difluorphenol, 4-Amino-2,5-difluorphenol, 4-Amino-2,6-difluorphenol, 4-Amino-2-fluorphenol, 4-Amino-3-chlor-5-fluorphenol, 4-Amino-3-chlor-2-fluorphenol, 4-Amino-5-chlor-2-fluorphenol, 4-Amino-3-brom-5-fluorphenol, 4-Amino-2-(trifluormethoxy)phenol und 4-Amino-3-(trifluormethoxy)-phenol.

Viele der Verbindungen der Formel (I) sind neu und ebenfalls von der Erfindung umfasst, wobei jedoch ausgenommen sind: 4-Amino-3,5-difluorphenol, 4-Amino-2,5-difluorphenol, 4-Amino-2,6-difluorphenol, 4-Amino-2-chlor-6-fluorphenol, 4-Amino-2-chlor-3-fluorphenol, 4-Amino-2-chlor-5-fluorphenol, 4-Amino-2-brom-5-fluorphenol, 4-Amino-2-fluorphenol, 4-Amino-3-fluorphenol, 4-Amino-2-(trifluor-methyl)phenol, 4-Amino-5-chlor-2-(trifluormethyl)phenol, 4-Amino-2-chlor-6-(trifluormethyl)-phenol, 4-Amino-3-(trifluormethyl)phenol.

Als besonders bevorzugte Einzelverbindungen der Formel (I) seien genannt:

4-Amino-2,3-difluorphenol, 4-Amino-3-chlor-5-fluorphenol, 4-Amino-3-chlor-2-fluorphenol, 4-Amino-5-chlor-2-fluorphenol, 4-Amino-3-brom-5-fluorphenol, 4-Amino-2-(trifluormethoxy)phenol und 4-Amino-3-(trifluormethoxy)phenol.

Gemäß Schritt A1) können die Verbindungen der Formel (III) aus Verbindungen der Formel (II) in an sich bekannter Weise hergestellt werden. Vorteilhafterweise werden die Verbindungen der Formel (II) in Gegenwart von Wasser und Säure mit einer Nitritquelle umgesetzt werden.

Als Säuren können beispielsweise Mineralsäuren wie Salzsäure, Bromwasserstoffsäure, Schwefelsäure oder Tetrafluoroborsäure sowie organische Sulfonsäuren verwendet werden.

Als Nitritquelle können beispielsweise die Alkalimetallnitrite wie insbesondere Natriumnitrit oder Kaliumnitrit, sowie organische Nitrite wie insbesondere tert.-Butylnitrit oder Methylnitrit eingesetzt werden. Bevorzugt sind Alkalimetallnitrite, die vorzugsweise in Wasser gelöst eingesetzt werden.

Das molare Verhältnis von Protonen der eingesetzten Säure zu Verbindungen der Formel (II) kann beispielsweise 1 bis 15 betragen, bevorzugt 2 bis 10 und besonders bevorzugt 2,5 bis 6,5.

Das molare Verhältnis von Verbindungen der Formel (II) zur Nitritquelle kann beispielsweise 0,8 bis 4 betragen, bevorzugt 1,0 bis 2,5 und besonders bevorzugt 1,05 bis 1,5.

Die Reaktionstemperatur im Schritt A1) kann beispielsweise -20°C bis 40°C betragen, bevorzugt -10°C bis 20°C und besonders bevorzugt -5°C bis 10°C, der Reaktionsdruck kann beispielsweise 0,5 bis 100 bar betragen, Umgebungsdruck ist bevorzugt, die Reaktionsdauer kann 10 min bis 5 Stunden betragen, bevorzugt sind 1 Stunde bis 3 Stunden.

Zur Umsetzung der Verbindungen der Formel (II) geht man beispielsweise so vor, dass die Verbindungen der Formel (II) in Wasser und Säure vorgelegt werden und anschließend die Zugabe einer wässrigen Lösung des Alkalimetallnitrits erfolgt. Nach beendeter Reaktionszeit kann ein Überschuss an Alkalimetallnitrit durch Zugabe einer primären Aminoverbindung, wie beispielsweise Harnstoff oder Amidoschwefelsäure vernichtet werden.

Die für Schritt A1) benötigten Ausgangsverbindungen der Formel (II) sind literaturbekannt oder analog zur Literatur synthetisierbar.

Gemäß Schritt A2) werden die in Schritt A1) erhaltenen Verbindungen der Formel (III) mit Verbindungen der Formel (IV) in Gegenwart von Base zu Verbindungen der Formel (V) umgesetzt.

Als Base eignen sich prinzipiell alle Basen, die basischer sind als die Verbindungen der Formel (IV), vorzugsweise um zumindest 2 pK-Einheiten.

Bevorzugt ist der Einsatz von Alkali- oder Erdalkalimetallhydroxiden, -carbonaten und -hydrogencarbonaten, wobei Alkalimetallhydroxide, wie insbesondere Natriumhydroxid bevorzugt sind.

Dabei kann die Base entweder vorgelegt werden oder im Verlauf der Reaktion in derart zugegeben werden, dass das Reaktionsmedium alkalisch bleibt.

Das molare Verhältnis von Verbindungen der Formel (III) zu Verbindungen der Formel (IV) kann beispielsweise 0,3 bis 5 betragen, bevorzugt 0,5 bis 2 und besonders bevorzugt 0,6 bis 0,9.

Das molare Verhältnis von Verbindungen der Formel (III) zu Base kann beispielsweise 1 bis 15, bevorzugt 3 bis 10 und besonders bevorzugt 4 bis 7 betragen.

Die Reaktionstemperatur in Schritt A2) kann beispielsweise -20°C bis 50°C betragen, bevorzugt 0° bis 40°C und besonders bevorzugt 5° bis 30°C, der Reaktionsdruck beispielsweise 0,5 bis 100 bar, wobei Umgebungsdruck bevorzugt ist. Weiterhin kann die Reaktionsdauer beispielsweise 10 min bis 15 Stunden betragen, bevorzugt sind 3 bis 5 Stunden.

Die Aufarbeitung der Verbindungen der Formel (V) kann in an sich bekannter Weise durch Extraktion und anschließende Destillation oder bei 30°C festen Verbindungen der Formel (V) durch Filtration und Kristallisation erfolgen.

Die Verbindungen der Formel (V) sind als wichtige Intermediate von der Erfindung ebenfalls umfasst, wobei jedoch 3,5-Difluor-4-phenylazo-phenol, 3-Fluor-4-phenylazo-phenol, 3-Fluor-4-(4'-nitrophenylazo)-phenol, 3-Fluor-4-(3'-nitrophenylazo)-phenol, 3-Fluor-4-(4'-thiocyanatophenylazo)-phenol, 3-Fluor-4-(4'-sulfo-phenylazo)-phenol, 4-(2'-Fluor-4'-hydroxyphenylazo)benzoesäureethylester, 2-Fluor-4-(4'-fluorphenylazo)-phenol, 2-Fluor-4-(3'-fluorphenylazo)-phenol, 2-Fluor-4-(4'-sulfophenylazo)-phenol, 4-(3'-Fluor-4'-hydroxyphenylazo)benzoesäureethylester, 2,3-Difluor-4-(4'-iodphenylazo)-phenol, 2,3-Difluor-4-(4'-sulfophenylazo)-phenol und 2,6-Difluor-4-(2'-bromphenylazo)-phenol, 3-(Trifluormethyl)-4-(phenylazo)-phenol, 3-(Trifluormethyl)-4-(4'-natriumsulfonatphenylazo)-phenol ausgenommen sind.

Die Verbindungen der Formel (IV) sind literaturbekannt oder analog der Literatur synthetisierbar.

Gemäß Schritt B) werden Verbindungen der Formel (V) durch Reduktion in Verbindungen der Formel (I) überführt.

Die Reduktion kann beispielsweise durch Reduktionsmittel, wie Natriumbisulfit, Titan(III)chlorid oder Zinn und Salzsäure erfolgen. Vorteilhafterweise wird die Reduktion in Gegenwart von Wasserstoff und Katalysator durchgeführt.

Bevorzugte Katalysatoren sind beispielsweise Metalle oder Metallverbindungen wie insbesondere Salze oder Komplexe von Nickel, Palladium, Platin, Cobalt, Rhodium, Iridium und Ruthenium, wobei Metalle wie Nickel oder Palladium bevorzugt sind. Vorzugsweise werden Metalle in fein verteilter Form wie zum Beispiel als Raney-Metall oder auf ein Trägermaterial aufgebracht verwendet.

Besonders bevorzugt wird die Reduktion mit Wasserstoff und Palladium auf Kohle durchgeführt.

Die Reduktion gemäß Schritt B) kann beispielsweise bei einer Reaktionstemperatur von 0°C bis 200°C, bevorzugt bei 10°C bis 80°C und besonders bevorzugt bei 20°C bis 40°C durchgeführt werden.

Der Wasserstoffpartialdruck kann bei der Reduktion beispielsweise 0,1 bis 180 bar betragen, bevorzugt 0,5 bis 50 bar und besonders bevorzugt 1 bis 3 bar.

Gegebenenfalls und vorzugsweise kann die Reduktion in Gegenwart von Lösungsmitteln durchgeführt werden, sofern sie im Wesentlichen unter den gewählten Reduktionsbedingungen inert sind.

Geeignete Lösungsmittel sind beispielsweise aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Alkohole wie beispielsweise Methanol, Ethanol und iso-Propanol oder Mischungen von Lösungsmitteln.

In einer besonders bevorzugten Ausführungsform wird die Reduktion in Gegenwart von Palladium auf Aktivkohle und in Gegenwart von Methanol oder Ethanol bei einem Wasserstoffpartialdruck von 1 bis 3 bar durchgeführt.

Die Reaktionsdauer bei der Reduktion kann 10 min bis 100 Stunden betragen, bevorzugt sind 2 bis 20 Stunden.

Die Aufarbeitung der Verbindungen der Formel (I) in der R' für Wasserstoff steht, kann in an sich bekannter Weise durch Extraktion und anschließende Destillation oder bei 30°C festen Verbindungen der Formel (I) durch Umkristallisation erfolgen.

Gegebenenfalls wird Schritt C), die O-Alkylierung von Verbindungen der Formel (I) in denen R¹ für Wasserstoff steht, vorgenommen.

Vorzugsweise wird dabei die Reaktion in Gegenwart von anorganischer Base durchgeführt.

Als anorganische Base eignen sich prinzipiell alle Basen, die basischer sind als die Verbindungen der Formel (I), in denen R¹ für Wasserstoff steht, vorzugsweise um zumindest 3 pK-Einheiten.

Bevorzugte anorganische Basen sind Alkalimetall- oder Erdalkalimetall-carbonate, -hydrogen-carbonate und -hydroxide sowie Tetraalkylammonium-hydroxide, wobei Alkalimetall-carbonate und -hydroxide bevorzugt sind.

Als Alkylierungsmittel eignen sich insbesondere Verbindungen der Formel (VI),

R¹-Akt (VI)

in der
- R¹: für C₁-C₁₂-Alkyl, C₅-C₁₅-Arylalkyl steht und
- Akt: für Iod, Brom, Chlor oder ein Sulfonat
steht.

Als Lösungsmittel zur Durchführung der Alkylierung eignen sich insbesondere Ether, wie Methyl-tert.-butylether, Dioxan, Tetrahydrofuran oder Ethylenglykoldimethyl- oder -diethylether; Amide, wie N,N-Dimethylformamid, N,N-Dimethylacetamid, N-Methyl-formanilid, N-Methyl-pyrrolidon, N-Methylcaprolactam oder Hexamethylphosphorsäuretriamid; Sulfoxide, wie Dimethylsulfoxid, Sulfone wie Tetramethylensulfon oder Gemische solcher organischen Lösungsmittel.

Das molare Verhältnis von anorganischer Base zu Verbindungen der Formel (I), in denen R¹ für Wasserstoff steht, kann beispielsweise 0,3 bis 5, bevorzugt 0,5 bis 3 und besonders bevorzugt 0,9 bis 1,3 betragen.

Das molare Verhältnis von Alkylierungsmittel zu Verbindungen der Formel (I), in denen R¹ für Wasserstoff steht, kann beispielsweise 0,3 bis 5, bevorzugt 0,5 bis 3 und besonders bevorzugt 0,9 bis 1,8 betragen.

Die Reaktionstemperatur in Schritt C) kann beispielsweise -70°C bis 100°C, bevorzugt -20 bis 60°C und besonders bevorzugt 0 bis 40°C betragen.

Die Reaktionsdauer in Schritt C) kann beispielsweise 10 min bis 24 Stunden betragen, bevorzugt sind 1 bis 8 Stunden.

Auf erfindungsgemäße Weise werden die Verbindungen der Formel (I), (VIII) und (X) in guten Ausbeuten ausgehend von einfach erhältlichen Edukten gewonnen. Die Verbindungen der Formel (I), (VIII) und (X), sowie die Verbindungen der Formel (V) eignen sich insbesondere zur Anwendung in einem Verfahren zur Herstellung von Wirkstoffen in Arzneimitteln und Agrochemikalien oder Zwischenprodukten davon.

Bevorzugte Wirkstoffe von Arzneimittel sind dabei solche, die zur Behandlung von chronischer Bronchitis eingesetzt werden, wobei solche bevorzugt sind, die in WO 03/08413 und PCT 03/02154 beschrieben sind.

### Beispiele

### Beispiel 1

### Herstellung von 3,5-Difluor-4-(phenyldiazenyl)phenol

Bei 0°C werden 108,2 g (1,16 mol) Anilin in 500 g 27 %iger Salzsäure unter heftigem Rühren gelöst. Danach werden bei 5° - 0°C 95,2 g (1,38 mol) Natriumnitrit (in 283 ml Wasser gelöst) zugetropft und die Lösung nach beendeter Zugabe noch 60 Minuten nachgerührt. Überschüssiges Natriumnitrit wird mit Amidoschwefelsäure vernichtet.

In einem weiteren Kolben werden 101,2 g (0,77 mol) 3,5-Difluorphenol in einer Lösung von 182,8 g (4,57 mol) Natriumhydroxid in 2281 ml Wasser gelöst und auf 5°C vorgekühlt. Zu dieser Lösung wird die oben frisch hergestellte Diazoniumsalz-Lösung so schnell zugegeben, dass die Innentemperatur nicht über 5°C steigt. Während der Zugabe wird der pH-Wert regelmäßig kontrolliert. Nach beendeter Zugabe lässt man auf Raumtemperatur aufwärmen, rührt 60 Minuten nach und stellt die Suspension dann mit 2N HCl auf pH 4 ein. Das Produkt wird abfiltriert und mehrmals mit Wasser gewaschen. Nach der Trocknung im Vakuumtrockenschrank erhält man 182 g (quantitative Ausbeute) der gewünschten orange-braunen Azoverbindung.
MS-EI: m/z 234 [M]⁺

### Beispiel 2

### Herstellung von 4-Amino-3,5-difluorphenol

20,4 g (0,09 mol) 3,5-Difluor-4-(phenyldiazenyl)phenol aus Beispiel 1 und 0,5 g Pd/C (10% ig) werden in 120 ml Methanol vorgelegt. Die Lösung wird entgast und mit Wasserstoff begast. Es wird für 16 Stunden bei Raumtemperatur unter 1 bar Wasserstoffatmosphäre gerührt. Nach beendeter Wasserstoffaufnahme wird der Katalysator abfiltriert und das Filtrat am Rotationsverdampfer eingeengt. Der Rückstand wird an Kieselgel (n-Hexan/Essigsäureethylester, 3:1) chromatographiert. Der erhaltene Feststoff wird mit n-Hexan verrührt und getrocknet. Man erhält 7,6 g (60 %) 4-Amino-3,5-difluorphenol als gelb-braunen Feststoff.
¹H NMR (400 MHz, CDCl₃): δ = 3,40 (s, 2 H, NH₂); 4,55 (s, 1 H, OH); 6.39 (m, 2 H, Ar-H)
¹⁹F NMR (376,3 MHz, CDCl₃): δ = -130,5
MS-EI: m/z 145 [M]⁺
Smp.: 150-151°C

### Beispiel 3

### Herstellung von 2,3-Difluor-4-(phenyldiazenyl)phenol

Bei 0°C werden 27,3 ml (0,3 mol) Anilin in 140 ml HCl (halbkonz.) unter heftigem Rühren eingetropft. Danach werden bei 5° - 0°C 21,7 g (0,315 mol) NaNO₂ (in 100 ml Wasser gelöst) zugetropft und die Lösung nach beendeter Zugabe noch 20 Minuten nachgerührt.

In einem weiteren Kolben werden 39 g (0,3 mol) 2,3-Difluorphenol in 300 ml 2N NaOH gelöst und auf 0°C vorgekühlt. Zu dieser Lösung wird die oben frisch hergestellte Diazolösung so schnell zugegeben, dass die Innentemperatur nicht über 5°C steigt. Während der Zugabe wird der pH-Wert regelmäßig kontrolliert und falls nötig mittels Na₂CO₃ wieder alkalisch gestellt. Nach beendeter Zugabe lässt man auf Raumtemperatur aufwärmen, rührt 30 Minuten nach und stellt die Suspension dann mit 2N HCl auf pH 3-4 ein. Das Produkt wird abfiltriert und mehrmals mit Wasser gewaschen. Nach der Trocknung im Vakuumtrockenschrank erhält man 61g (87 %) der gewünschten hellgelben Azoverbindung.
MS-EI: m/z 234 [M]⁺

### Beispiel 4

### Herstellung von 4-Amino-2,3-difluorphenol

61 g (0,26 mol) 2,3-Difluor-4-(phenyldiazenyl)phenol aus Beispiel 3 und 1 g Pd/C (10 %ig) werden in 800 ml Ethanol vorgelegt. Die Lösung wird entgast und mit Wasserstoff begast. Es wird für 18 Stunden bei Raumtemperatur unter 1 bar Wasserstoffatmosphäre gerührt. Nach beendeter Wasserstoffaufnahme wird der Katalysator abfiltriert und das Filtrat am Rotationsverdampfer eingeengt, wobei das Produkt bereits auskristallisiert. Der erhaltene Feststoff wird abfiltriert, mit wenig kaltem Ethanol nachgewaschen und für 4 Stunden am Hochvakuum getrocknet. Man erhält 28 g (74 %) der erwünschten Verbindung als hellbraunes Pulver.
¹H NMR (400 MHz, DMSO-d₆) δ = 4.72 (s, 2H, NH₂), 6.39 (t, 1H, Ar-H), 6.51 (t, 1H, Ar-H), 9.13 (s, 1H, OH);
MS-EI: m/z 145 [M]⁺

### Beispiel 5

### Herstellung von 2,5-Difluor-4-(phenyldiazenyl)phenol

Bei 0°C werden 17,5 ml (0,19 mol) Anilin in 185 ml HCl (halbkonz.) unter heftigen Rühren eingetropft. Danach werden bei 5° - 0°C 13,9 g (0,2 mol) NaNO₂ (in 50 ml Wasser gelöst) zugetropft und die Lösung nach beendeter Zugabe noch 20 Minuten nachgerührt.

In einem weiteren Kolben werden 25 g (0,19 mol) 2,5-Difluorphenol in 190 ml 2N NaOH gelöst und auf 0°C vorgekühlt. Zu dieser Lösung wird die oben frisch hergestellte Diazolösung so schnell zugegeben, dass die Innentemperatur nicht über 5°C steigt. Während der Zugabe wird der pH-Wert regelmäßig kontrolliert und falls nötig mittels Na₂CO₃ wieder alkalisch gestellt. Nach beendeter Zugabe lässt man auf Raumtemperatur aufwärmen, rührt 30 Minuten nach und stellt die Suspension im Anschluss mit 2N HCl auf pH 3-4 ein. Das Produkt wird abfiltriert und mehrmals mit Wasser gewaschen. Nach Trocknung im Vakuumtrockenschrank bei 50°C erhält man 42 g (93 %) der gewünschten orangefarbenen Azoverbindung.
MS-EI: m/z 234 [M]⁺

### Beispiel 6

### Herstellung von 4-Amino-2,5-difluorphenol

42 g (0,18 mol) 2,5-Difluor-4-(phenyldiazenyl)phenol aus Beispiel 5 und 1 g Pd/C (10 %ig) werden in 650 ml Ethanol vorgelegt. Die Lösung wird entgast und mit Wasserstoff begast. Es wird für 18 Stunden bei Raumtemperatur unter 1 bar Wasserstoffatmosphäre gerührt. Nach beendeter Wasserstoffaufnahme wird der Katalysator abfiltriert und das Filtrat im Vakuum destilliert und bei 70°C für 4 Stunden am Hochvakuum getrocknet. Der Rückstand (24 g) wird aus Ethanol umkristallisiert und getrocknet. Man erhält 15 g (60 %) 4-Amino-2,5-difluorphenol als orangebraunes Pulver.
¹H NMR (400 MHz, DMSO-d₆) δ = 4,68 (s, 2H, NH₂), 6,58 (m, 2H, Ar-H), 9,05 (s, 1H, OH);
MS-EI: m/z 145 [M]⁺

### Beispiel 7

### Herstellung von 2-Trifluormethoxy-4-(phenyldiazenyl)phenol

Bei 0°C werden 10,23 ml (0,11 mmol) Anilin in 50 ml HCl (halbkonz.) unter heftigen Rühren eingetropft. Danach werden bei 5° - 0°C 8,1 g (0,117 mol) NaNO₂ (in 30 ml Wasser gelöst) zugetropft und die Lösung nach beendeter Zugabe noch 20 Minuten nachgerührt.

In einem weiteren Kolben werden 20,0 g (0,12 mol) 2-Trifluormethoxyphenol in 140 ml 2N NaOH gelöst und auf 0°C vorgekühlt. Zu dieser Lösung wird die oben frisch hergestellte Diazolösung so schnell zugegeben, dass die Innentemperatur nicht über 5°C steigt. Während der Zugabe wird der pH-Wert regelmäßig kontrolliert und falls nötig mittels Na₂CO₃ wieder alkalisch gestellt. Nach beendeter Zugabe lässt man auf Raumtemperatur aufwärmen, rührt 30 Minuten nach und stellt die Suspension dann mit 2N HCl auf pH 3-4 ein. Das Produkt wird abgesaugt und mehrmals mit Wasser gewaschen. Nach der Trocknung im Vakuumtrockenschrank erhält man 26g (81%) der gewünschten hellbraunen Azoverbindung.

### Beispiel 8

### Herstellung von 4-Amino-2-trifluormethoxyphenol

25 g (0,09 mol) 2-Trifluormethoxy-4-(phenyldiazenyl)phenol aus Beispiel 7 und 1 g Pd/C (10 %ig) werden in 350 ml Ethanol vorgelegt. Die Lösung wird entgast und mit Wasserstoff begast. Es wird für 72 Stunden bei Raumtemperatur unter 1 bar Wasserstoffatmosphäre gerührt. Nach beendeter Wasserstoffaufnahme wird der Katalysator abfiltriert und das Filtrat am Rotationsverdampfer eingeengt, wobei das Produkt bereits auskristallisiert. Der erhaltene Feststoff wird abgesaugt, mit wenig kaltem Ethanol nachgewaschen und für 4 Stunden am Hochvakuum getrocknet. Man erhält 8,5 g (50 %) der erwünschten Verbindung als hellbraunes Pulver.
¹H NMR (400 MHz, DMSO-d₆) δ = 4.72 (s, 2H, NH₂), 6.42 (d, 1H, J = 8.6 Hz, Ar-H), 6.5 (s, 1H, Ar-H), 6.72 (d, 1H, J = 8.6 Hz, Ar-H), 8.9 (s, 1H, OH);
MS-EI: m/z 193 [M]⁺

## Patentansprüche

1. Verfahren zur Herstellung von Verbindungen der Formel (I), 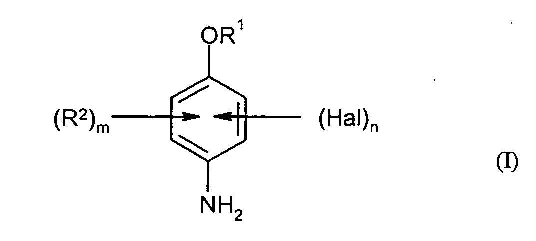 in der
m für 0, 1, 2 oder 3 steht und
n für 1, 2, 3 oder 4 steht,
wobei die Summe aus
m + n maximal 4 ist und
R¹ für Wasserstoff, C₁-C₁₂-Alkyl oder C₅-C₁₅-Arylalkyl und
R² jeweils unabhängig für C₁-C₁₂-Fluoralkyl, C₁-C₁₂-Fluoralkylthio oder C₁-C₁₂-Fluoralkoxy und
Hal jeweils unabhängig für Brom, Chlor oder Fluor steht
**dadurch gekennzeichnet, dass**
• in einem Schritt A1)
Verbindungen der Formel (II) 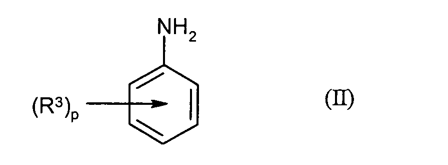 in der
p für 0, 1, 2 oder 3 steht und
R³ jeweils unabhängig voneinander für Fluor, Chlor, Brom, Iod, Cyano, Thiocyanato, Hydroxysulfonyl oder Alkalimetallsalze davon, Nitro, C₁-C₁₂-Alkyl, C₁-C₁₂-Fluoralkyl, C₁-C₁₂-Fluoralkyoxy, C₁-C₁₂-Fluoralkylthio, C₁-C₁₂-Alkoxy, C₁-C₁₂-Alkoxycarbonyl, Di(C₁-C₁₂-alkyl)amino, C₄-C₁₄-Aryl oder C₅-C₁₅-Arylalkyl steht,
in Diazoniumsalze der Formel (III) überführt werden 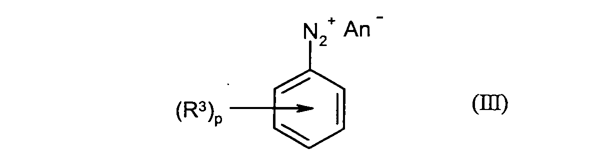 in der An⁻ für das Anion einer Säure steht
und
• in einem Schritt A2)
die Diazoniumsalze der Formel (III) mit Verbindungen der Formel (IV) 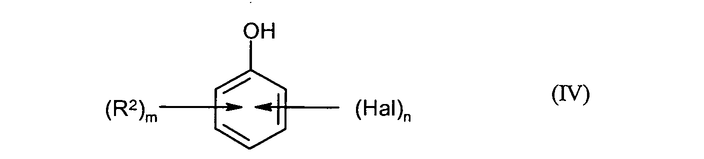 in der
m, n, Hal und R² die unter der Formel (I) angegebene Bedeutung besitzen
in Gegenwart von Base zu Verbindungen der Formel (V) umgesetzt werden 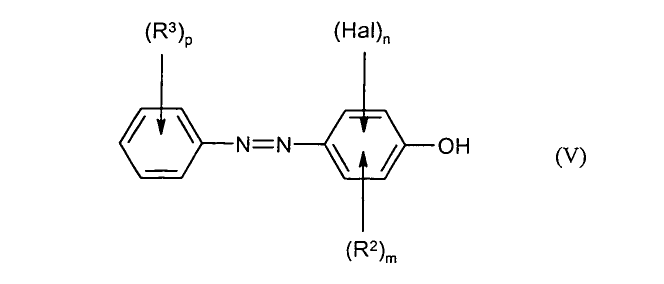 und
• in einem Schritt B) die Verbindungen der Formel (V) mit einem Reduktionsmittel in die Verbindungen der Formel (I) überführt werden, in der R¹ für Wasserstoff steht und
• gegebenenfalls in einem Schritt C) diese Verbindungen durch O-Alkylierung in Verbindungen der Formel (I) überführt werden, in der R¹ für C₁-C₁₂-Alkyl steht.

2. Verfahren nach Anspruch 1, **dadurch gekennzeichnet, dass** 4-Amino-2,3-difluorphenol, 4-Amino-2,5-difluorphenol, 4-Amino-2,6-difluorphenol, 4-Amino-2-fluorphenol, 4-Amino-3-chlor-5-fluorphenol, 4-Amino-3-chlor-2-fluorphenol, 4-Amino-5-chlor-2-fluorphenol, 4-Amino-3-brom-5-fluorphenol, 4-Amino-2-(trifluormethoxy)phenol und 4-Amino-3-(trifluormethoxy)-phenol hergestellt werden.

3. Verfahren nach einem oder mehreren der Ansprüche 1 und 2, **dadurch gekennzeichnet, dass** in einem weiteren Schritt D)
• die Verbindungen der Formel (I) durch Umsetzung mit Verbindungen der Formeln (VIIa) oder (VIIb) 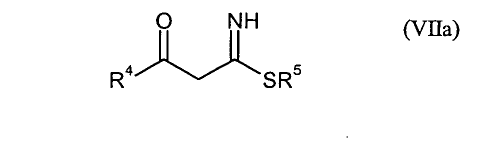 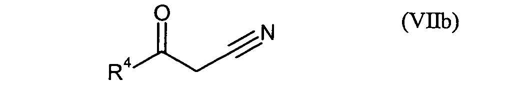 in denen
R⁴ für 2,4-Difluorphenyl, 4-Fluorphenyl, 2,3-Difluorphenyl oder 4-Fluor-3-chlorphenyl und
R⁵ für gegebenenfalls einfach oder mehrfach durch Chlor oder Fluor substituiertes Phenyl steht,
in Verbindungen der Formel (VIII) überführt werden, 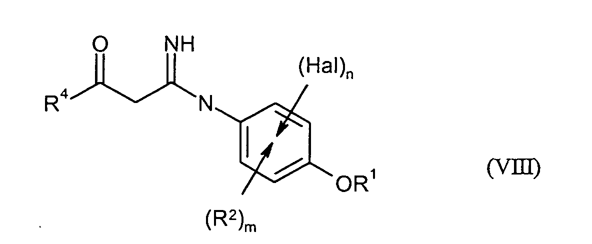 in der
R¹, R², R⁴, Hal, n und m die obenstehend oder in Anspruch 1 genannte Bedeutung besitzen.

4. Verfahren nach einem oder mehreren der Ansprüche 1 bis 3, **dadurch gekennzeichnet, dass** in einem weiteren Schritt E)
• die Verbindungen der Formel (VIII) durch Umsetzung mit Verbindungen der Formel (IX) 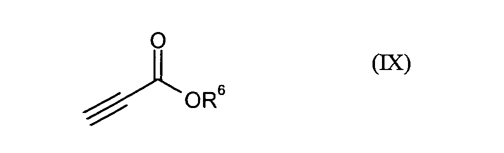 in der
R⁶ für C₁-C₁₂-Alkyl, C₅-C₁₅-Arylalkyl oder C₄-C₁₄-Aryl steht
in Verbindungen der Formel (X) überführt werden, 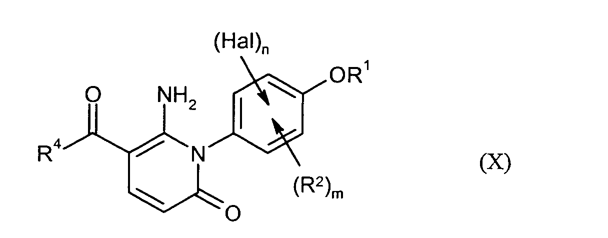 in der
R¹, R², R⁴, R⁶, Hal, n und m die oben oder in Anspruch 1 genannte Bedeutung besitzen.

5. Verbindungen der Formel (I) wobei ausgenommen sind: 4-Amino-3,5-difluorphenol, 4-Amino-2,5-difluorphenol, 4-Amino-2,6-difluorphenol, 4-Amino-2-chlor-6-fluorphenol, 4-Amino-2-chlor-3-fluorphenol, 4-Amino-2-chlor-5-fluorphenol, 4-Amino-2-brom-5-fluorphenol, 4-Amino-2-fluorphenol, 4-Amino-3-fluorphenol, 4-Amino-2-(trifluor-methyl)phenol, 4-Amino-5-chlor-2-(trifluormethyl)phenol, 4-Amino-2-chlor-6-(trifluormethyl)phenol und 4-Amino-3-(trifluormethyl)phenol.

6. Verbindungen der Formel (V) wobei ausgenommen sind: 3,5-Difluor-4-phenylazo-phenol, 3-Fluor-4-phenylazo-phenol, 3-Fluor-4-(4'-nitrophenylazo)-phenol, 3-Fluor-4-(3'-nitrophenylazo)-phenol, 3-Fluor-4-(4'-thiocyanatophenylazo)-phenol, 3-Fluor-4-(4'-sulfophenylazo)-phenol, 4-(2'-Fluor-4'-hydroxyphenylazo)benzoesäureethylester, 2-Fluor-4-(4'-fluorphenylazo)-phenol, 2-Fluor-4-(3'-fluorphenylazo)-phenol, 2-Fluor-4-(4'-sulfophenylazo)-phenol, 4-(3'-Fluor-4'-hydroxyphenylazo)benzoesäureethylester, 2,3-Difluor-4-(4'-iodphenylazo)-phenol, 2,3-Difluor-4-(4'-sulfophenylazo)-phenol und 2,6-Difluor-4-(2'-bromphenylazo)-phenol, 3-(Trifluormethyl)-4-(phenylazo)-phenol und 3-(Trifluormethyl)-4-(4'-natriumsulfonatphenylazo)-phenol.

7. Verwendung von Verbindungen gemäß den Ansprüchen 5 und 6 oder solchen, die gemäß einem Verfahren nach Anspruch 1 bis 4 hergestellt wurden, in einem Verfahren zur Herstellung von Wirkstoffen in Arzneimitteln und Agrochemikalien oder Zwischenprodukten davon.
